# FASCICULE DE BREVET EUROPEEN

(11) **EP 1 177 276 B1**
(45) Date de publication et mention de la délivrance du brevet: **13.10.2004**
(21) Numéro de dépôt: 00925408.7
(22) Date de dépôt: 05.05.2000
(51) Int. Cl.: C12M 1/33, C12M 3/08, B01J 19/00, G01N 1/28

(54) **Dispositif pour l'extraction d'ADN, d'ARN et de protéines à partir de prélèvements biologiques**
Vorrichtung zur Gewinnung von DNS, RNS und Proteinen aus biologischen Proben
Device for extracting DNA, RNA and proteins from biological samples

(30) Priorité: 05.05.1999 FR 9905795; 02.08.1999 FR 9910040
(43) Date de publication de la demande: 06.02.2002
(73) Titulaire: Genomic S.A., 74160 Collonges sous Saleve (FR)
(72) Inventeur: GAZEAU, Michel, F-74160 Collonges sous Saleve (FR)
(74) Mandataire: Breese, Pierre
(86) Numéro de dépôt international: PCT/FR2000/001230
(87) Numéro de publication internationale: WO 2000/068358

(56) Documents cités:
- EP-A- 0 123 316
- EP-A- 0 487 028
- WO-A-96/15576
- WO-A-98/20164

## Description

La présente invention concerne le domaine de l'extraction d'ADN, d'ARN et de protéines à haut débit, à partir de prélèvements biologiques non liquides ou de cellules en suspension, à l'état naturel ou entrant dans des préparations par exemple alimentaires.

On connaît dans l'état de la technique différents procédés d'extraction d'acides nucléiques et de protéines à partir de prélèvements mettent en jeu une première étape de broyage de l'échantillon, par des procédés divers tels pilage, ultrasons, haute pression ...

WO-A-98/20164 décrit un appareil pour l'extraction d'ADN d'une pluralité d'échantillons biologiques qui comprend une plaque multipuits qui est coincée entre deux plaques génératrices d'un champ éléctromagnétique variable. Chacun des puits contient un micropilon qui comprend au moins un centre d'un matériau ferreux. EP-A-0 487 028 décrit un appareil pour l'extraction et la purification automatique d'ADN d'un échantillon biologique qui comprend une plaque multipuits sur laquelle se glisse une file de seringues pour introduire et enlever l'échantillon succesivement dans une série de puits.

Il est intéressant, pour des échantillons mous, de les broyer à très basse température. En effet, cela diminue les risques de dégradation des macromolécules et facilite l'éclatement des cellules et donc améliore le rendement des extractions. Il s'en suit différents traitements de l'échantillon, qui visent à le laver, à supprimer les membranes cellulaires et à éliminer les produits non voulus. La première étape n'étant pas systématiquement mise en oeuvre. Un procédé d'extraction d'acides nucléiques et de protéines mis en oeuvre avec le dispositif qui fait l'objet de la demande de brevet comporte les étapes suivantes :

Il consiste, après broyage à basse température, (température inférieure à la température ambiante) par des procédés divers, simultanément, d'un nombre d'échantillons, à ajouter automatiquement et avec un temps d'attente minimal, un volume suffisamment précis de liquide, à chaque échantillon broyé, tout en contrôlant la température du broyat, du/des liquide/s et du mélange et à homogénéiser chaque mélange.

L'un des liquides peut de préférence être un tampon de lyse des parois cellulaires et plus précisément, un tampon de lyse dénaturant.

Il s'agit en particulier d'éviter que les agents de dégradation des macro-molécules puissent agir, tout en permettant l'écoulement du/des liquide/s, même si le broyage a eu lieu à très basse température (inférieure à 0°C)

Il s'agit de limiter les risques de contamination des personnes, par des virus, par exemple et de diminuer la perte de matériel biologique qui adhérerait aux pièces de broyage.

Il permet le traitement de plusieurs échantillons simultanément, et l'enchaînement des opérations et leur automatisation.

L'invention concerne en particulier le dispositif pour le broyage d'échantillons biologiques en vue de l'extraction d'ADN, d'ARN et de protéines caractérisé en ce qu'il comporte une pluralité de pilons rotatifs supportés par un bloc porte-pilons et un plateau mobile inférieur, mobile entre une position de travail rapprochée du bloc porte-pilons et une position de repos écartée du bloc porte-pilons, chacun des pilons comprenant au moins un conduit pour la distribution de liquide. Avantageusement, le plateau inférieur présente des moyens pour recevoir des tubes, disposés selon un arrangement conforme à celui des pilons ou une pluralité de cupules, disposés selon un arrangement conforme à celui des pilons. Selon une variante, le dispositif comprend en outre des moyens pour permettre l'écoulement du ou des liquides par gravité, lors de l'ouverture d'électrovannes.

Le dispositif préféré, décrit à titre d'exemple est constitué des éléments suivants :
- L'équipement de broyage de base, qui est dédié aux échantillons végétaux, pour lesquels seules les protéines comptent, auquel est ajouté :

- Un dispositif constitué d'un réseau de buses, chacune étant située au voisinage de la tige de chaque pilon, communicant par des cathéters avec le/les réservoir/s du/des liquide/s à distribuer. Les réservoirs peuvent être maintenus à des températures définies. Le/les liquides peuvent s'écouler par gravité, lors de l'ouverture d'électrovannes et le volume distribué réglé grâce à des cathéters de longueur et de diamètre précis, en jouant sur la durée d'ouverture des électrovannes. Une autre solution est par exemple d'aspirer et de refouler le/les liquides au moyen de pompes de précision. Dans ce cas il est facile de purger les cathéters. Les tiges de pilons peuvent servir de guide à l'écoulement du/des liquides vers les échantillons. Dans tous les cas, la distribution du liquide s'effectue sur un ou plusieurs sous-ensembles de tubes. Si l'on choisit des sous ensembles de 24 tubes parmi l'ensemble de 96 tubes, ou si une partie seulement des positions de broyage est occupée, il est possible de traiter seulement 24 échantillons de plus gros volume et d'éviter le gaspillage de liquide.
- Des dispositifs de chauffage régulés, qui peuvent être des résistances noyées dans le plateau porte tubes du Équipement de broyage et/ou des rampes à infrarouge disposées autour de l'espace de travail.
- Accessoirement, une plaque rigide, traversée par les tiges des pilons peut être rajoutée au dispositif préféré, pour couvrir chaque échantillon dans son conteneur, pendant le broyage, afin d'éviter des contaminations croisées. Cette plaque, grâce aux fraisages de chaque passage de tige de pilon sur la partie supérieure, favorise l'écoulement uniforme du/des liquides dans chaque conteneur d'échantillon, autour des pilons et contribue à les débarrasser des fragments adhérents.

L'automate programmable qui contrôle l'équipement de broyage contrôle également les écoulements de liquide, les températures et le verrouillage de la plaque rigide.

L'invention sera mieux comprise à la lecture de la description qui suit, se référant à un exemple non limitatif de réalisation et aux figures annexées où :

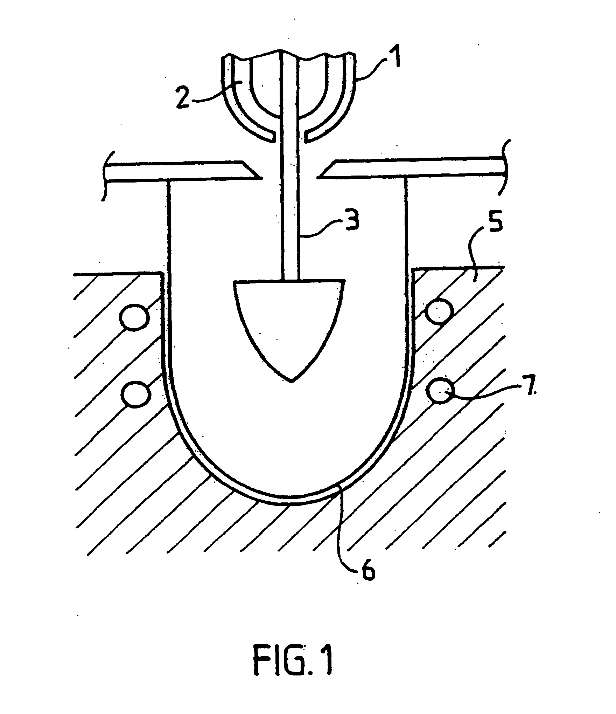

- la figure 1 représente une vue agrandie partielle d'un dispositif selon l'invention
- la figure 2 représente une vue en coupe d'une variante de réalisation
- la figure 3 représente une vue schématique du couvercle du dispositif représenté en figure 2.

Le dispositif est constitué par un automate formé par un châssis comportant deux blocs mobiles.

Le bloc supérieur est un plateau supportant une pluralité de porte-pilons (1) rotatifs, arrangés pour former une matrice de 96 têtes par exemple. Les pilons sont interchangeables et peuvent présenter une variété de taille ou de forme. La vitesse de rotation des pilons est comprise entre 100 et 1100 tours par minutes. Un programme de commande permet de déterminer le temps, la vitesse, le sens de rotation, la pression et les différents paramètres du processus de broyage souhaité. L'équipement comporte une mémoire pour l'enregistrement de plusieurs profils de broyage.

L'automate réalise les fonctions suivantes :
- prise de pilons
- lavage et décontamination des pilons
- mise en température des pilons
- broyage d'échantillons mous
- broyage de grains
- broyages spéciaux
- extraction des pilons.

Il comporte également un détecteur inactivant le dispositif lorsque l'opérateur accède à l'espace de travail comprenant les plateaux supérieur et inférieur.

Le bloc inférieur (5) est un plateau mobile qui supporte des racks interchangeables, présentant des moyens pour recevoir des tubes remplis d'échantillons à broyer, ou des cupules pour recevoir directement des matériaux biologiques tels que des graines.

Les tubes sont maintenus dans des supports transparents de 6 tubes. Quatre supports de 6 tubes occupent chacun la surface d'une microplaque, ce qui permet de centrifuger 96 tubes à la fois sur un rotor approprié. Dans le cas du broyage de graines, le plateau se présente sous la forme d'un ensemble de cupules en acier inoxydable dur dont le fond peut être ouvert ou fermé, et les pilons présentent plusieurs facettes. Le produit du broyage est alors récupéré dans des tubes placés sous les cupules.

Le bloc inférieur est mobile verticalement pour assurer la pénétration des pilons dans les tubes (6) ou cupules, ou au contraire écarter les pilons pour charger ou décharger l'équipement. Le plateau inférieur (5) est thermostaté, par exemple par des conduits (7) pour la circulation d'un fluide de refroidissement.

Un plateau couvercle additionnel peut être disposé sur le plateau porte-tube.

Les différents blocs sont amovibles et peuvent être extraits de l'équipement par un simple glissement vers l'avant.

Le porte-pilon (1) présente un conduit (2) d'arrivée de liquide. Le liquide vient ruisseler le long de la tige (3) du pilon jusqu'à la tête du pilon.

Les pilons peuvent être lavés et essorés automatiquement, par l'injection de liquides grâce aux conduits (2) ou en les faisant tourner à grande vitesse.

Lorsque les pilons sortent des tubes (6), ils sont parfaitement immobiles car c'est le plateau porte-tube qui s'écarte en descendant. Ce mode de fonctionnement évite la dissémination de matériel biologique servant de substrat à une analyse par PCR.

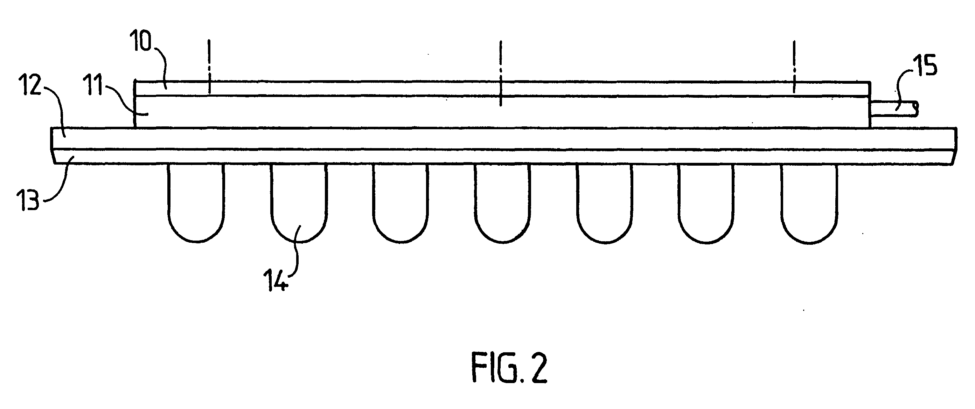

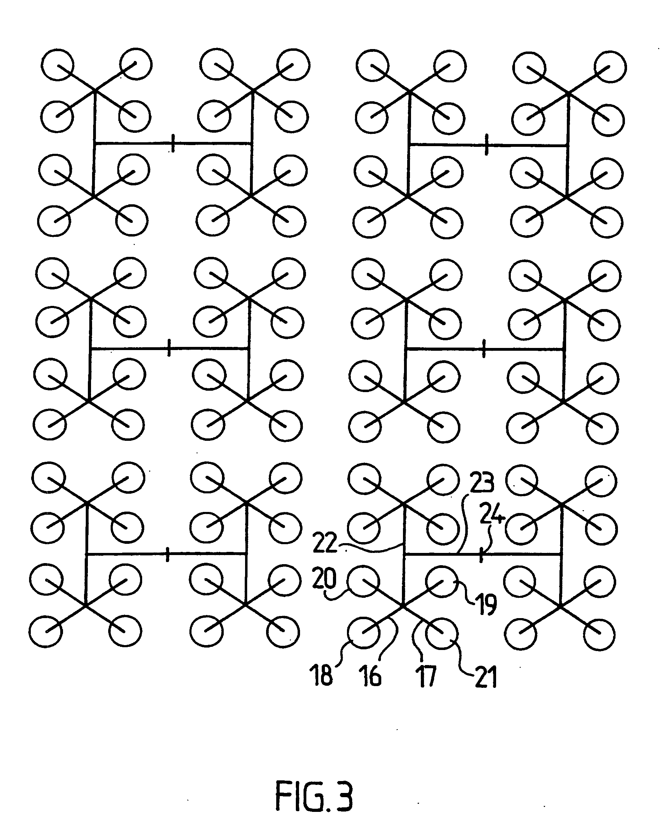

La figure 2 représente une vue en coupe d'une variante de réalisation et la figure 3 représente une vue schématique du plateau couvercle dudit dispositif.

Le plateau couvercle est composé d'une superposition de plateaux (10 à 13). Il vient coiffer les tubes (14) pour l'alimentation des tubes en réactifs et tampons.

Il comporte un plateau supérieur (10) en acier ou aluminium et deux plateaux intercalaires (11, 12) en téflon ainsi qu'un plateau inférieur (13) en silicone assurant l'étanchéité des tubes (14). Le plateau inférieur (13) en silicone est percé de trous correspondant aux ouvertures des tubes.

Les plateaux intercalaires (11, 12) présentent des canaux fraisés comme représenté en figure 3. Ces canaux forment un réseau pour la répartition des fluides provenant d'un conduit d'alimentation (15) jusqu'au niveau de l'ouverture de chacun des tubes.

Ces canaux forment dans le plateau (12) des séries de croisillons constitués de deux bras (16, 17) reliant chacun deux ouvertures respectivement (18, 19), (20, 21).

Ces croisillons sont reliés deux à deux par des bras de liaison (22) qui joignent la zone d'intersection des bras de deux croisillons adjacents.

Deux bras de liaison (22) sont eux même reliés par des bras collecteurs (23), reliant le milieu des deux bras de liaison.

Ces bras collecteurs (23) débouchent dans un réseau d'alimentation formé de rainures pratiquées dans le plateau (11), pour déboucher finalement dans le conduit d'alimentation (15).

Les bras sont déterminés de façon à ce que les trajets aboutissant aux différents tubes soient identiques. Les raccordements entre les différents bras sont symétriques.

## Revendications

1. Dispositif pour le broyage d'échantillons biologiques en vue de l'extraction d'ADN, d'ARN et de protéines **caractérisé en ce qu'**il comporte une pluralité de pilons rotatifs supportés par un bloc porte-pilons (1) et un plateau mobile inférieur (5), mobile entre une position de travail rapprochée du bloc porte-pilons (1) et une position de repos écartée du bloc porte-pilons (1), chacun des pilons comprenant au moins un conduit pour la distribution de liquide.

2. Dispositif pour le broyage d'échantillons biologiques en vue de l'extraction d'ADN, d'ARN et de protéines selon la revendication 1 **caractérisé en ce que** le plateau inférieur (5) présente des moyens pour recevoir des tubes (6), disposés selon un arrangement conforme à celui des pilons.

3. Dispositif pour le broyage d'échantillons biologiques en vue de l'extraction d'ADN, d'ARN et de protéines selon la revendication 1 **caractérisé en ce que** le plateau inférieur (5) présente une pluralité de cupules, disposés selon un arrangement conforme à celui des pilons.

4. Dispositif pour l'extraction d'ADN, d'ARN et de protéines à partir de prélèvements biologiques selon la revendication 1, 2 ou 3, **caractérisé en ce qu'**il comprend en outre des moyens pour permettre l'écoulement du ou des liquides par gravité, lors de l'ouverture d'électrovannes.

5. Dispositif pour l'extraction d'ADN, d'ARN et de protéines à partir de prélèvements biologiques selon la revendication 1, 2 ou 3, **caractérisé en ce qu'**il comprend des pompes et des moyens de purge des cathéters.

6. Dispositif pour l'extraction d'ADN, d'ARN et de protéines à partir de prélèvements biologiques selon la revendication 3, **caractérisé en ce que** le plateau inférieur (5) comporte des dispositifs de chauffage régulés.

7. Dispositif pour l'extraction d'ADN, d'ARN et de protéines à partir de prélèvements biologiques selon la revendication 1 **caractérisé en ce qu'**il en outre une plaque rigide, traversée par les tiges des pilons pour couvrir chaque échantillon dans son conteneur, pendant le broyage, afin d'éviter des contaminations croisées.

8. Dispositif pour l'extraction d'ADN, d'ARN et de protéines à partir de prélèvements biologiques selon la revendication 1 **caractérisé en ce qu'**il en outre un plateau couvercle composé d'une superposition de plateaux (10 à 13), les plateaux intercalaires (11, 12) présentant un réseau de fraisage aboutissant à des orifices pour l'alimentation des tubes (14).

9. Dispositif pour l'extraction d'ADN, d'ARN et de protéines à partir de prélèvements biologiques selon la revendication 8 **caractérisé en ce que** le couvercle comporte un plateau supérieur (10) et deux plateaux intercalaires (11, 12) en téflon ainsi qu'un plateau inférieur (13) en silicone assurant l'étanchéité des tubes (14).

10. Dispositif pour l'extraction d'ADN, d'ARN et de protéines à partir de prélèvements biologiques selon la revendication 9 **caractérisé en ce que** les fraisages sont des canaux formant un réseau pour la répartition des fluides provenant d'un conduit d'alimentation (15) jusqu'au niveau de l'ouverture de chacun des tubes.

11. Dispositif pour l'extraction d'ADN, d'ARN et de protéines à partir de prélèvements biologiques selon la revendication 10 **caractérisé en ce que** les canaux forment dans le plateau (12) des séries de croisillons constitués de deux bras (16, 17) reliant chacun deux ouvertures respectivement (18, 19), (20, 21), ces croisillons étant reliés deux à deux par des bras de liaison (22) qui joignent la zone d'intersection des bras de deux croisillons adjacents, deux bras de liaison (22) étant eux-même reliés par des bras collecteurs (23), reliant le milieu des deux bras de liaison.

## Patentansprüche

1. Vorrichtung zum Zermahlen von biologischen Mustern zur Extraktion von DNS, von RNS und von Proteinen, **dadurch gekennzeichnet, dass** ebendiese Vorrichtung eine Vielzahl von drehbaren Stößeln aufweist, wobei diese Stößel von einem Stößelhalterblock (1) und einer unteren mobilen Platte (5) getragen werden, wobei die letzte zwischen einer dem Stößelhalterblock (1) angenäherten Arbeitsposition und einer vom Stößelhalterblock (1) entfernten Ruheposition mobil ist, und wobei jeder der Stößel mindestens eine Leitung für die Verteilung von einer Flüssigkeit aufweist.

2. Vorrichtung zum Zermahlen von biologischen Mustern zur Extraktion von DNS, von RNS und von Proteinen nach Anspruch 1, **dadurch gekennzeichnet, dass** die untere Platte (5) Mittel zur Aufnahme der Rohre (6) aufweist, die nach einer Anordnung angeordnet sind, die derjenigen von den Stößeln entspricht.

3. Vorrichtung zum Zermahlen von biologischen Mustern zur Extraktion von DNS, von RNS und von Proteinen nach Anspruch 1, **dadurch gekennzeichnet, dass** die untere Platte (5) eine Vielzahl an Bechern aufweist, die nach einer Anordnung angeordnet sind, die derjenigen von den Stößeln entspricht.

4. Vorrichtung zum Zermahlen von biologischen Mustern zur Extraktion von DNS, von RNS und von Proteinen anhand von biologischen Proben nach einem der Ansprüche 1, 2 oder 3, **dadurch gekennzeichnet, dass** sie weiterhin Mittel aufweist, um den Gravitationsausfluss von der oder den Flüssigkeiten zu ermöglichen, wenn sich die Magnetschieber öffnen.

5. Vorrichtung zum Zermahlen von biologischen Mustern zur Extraktion von DNS, von RNS und von Proteinen anhand von biologischen Proben nach einem der Ansprüche 1, 2 oder 3, **dadurch gekennzeichnet, dass** sie weiterhin Pumpen und Mittel zur Entleerung der Katheter aufweist.

6. Vorrichtung zum Zermahlen von biologischen Mustern zur Extraktion von DNS, von RNS und von Proteinen anhand von biologischen Proben nach Anspruch 3, **dadurch gekennzeichnet, dass** die untere Platte (5) geregelte Heizeinrichtungen aufweist.

7. Vorrichtung zum Zermahlen von biologischen Mustern zur Extraktion von DNS, von RNS und von Proteinen anhand von biologischen Proben nach Anspruch 1, **dadurch gekennzeichnet, dass** sie weiterhin eine feste Platte aufweist, die von den Stäben der Stößel durchstoßen wird, um jedes Muster in seinem Behälter während des Zermahlvorgangs zu decken, um Kreuzkontaminierungen zu vermeiden

8. Vorrichtung zum Zermahlen von biologischen Mustern zur Extraktion von DNS, von RNS und von Proteinen anhand von biologischen Proben nach Anspruch 1, **dadurch gekennzeichnet, dass** sie weiterhin eine Deckelplatte bestehend aus einer Überlagerung von Platten (10 bis 13) aufweist, wobei die Zwischenplatten (11, 12) über ein Fräsennetz verfügen, das zur Versorgung der Rohre (14) in Öffnungen mündet.

9. Vorrichtung zum Zermahlen von biologischen Mustern zur Extraktion von DNS, von RNS und von Proteinen anhand von biologischen Proben nach Anspruch 8, **dadurch gekennzeichnet, dass** der Deckel eine obere Platte (10) und zwei Zwischenplatten (11, 12) aus Teflon sowie eine untere Platte (13) aus Silikon aufweist, um die Dichtheit der Rohre (14) zu sichern.

10. Vorrichtung zum Zermahlen von biologischen Mustern zur Extraktion von DNS, von RNS und von Proteinen anhand von biologischen Proben nach Anspruch 9, **dadurch gekennzeichnet, dass** die Fräsen ein Netz bildende Kanäle sind, und dies zur Verteilung der Flüssigkeiten, die aus einer Versorgungsleitung (15) stammen bis zum Öffnungsniveau von jedem Rohr.

11. Vorrichtung zum Zermahlen von biologischen Mustern zur Extraktion von DNS, von RNS und von Proteinen anhand von biologischen Proben nach Anspruch 10, **dadurch gekennzeichnet, dass** die Kanäle in der Platte (12) Serien an Kreuzen bestehend aus zwei Armen (16, 17), die jeweils zwei Öffnungen (18, 19), (20, 21) miteinander verbinden, bilden, wobei diese Kreuze jeweils zu zweit über Verbindungsarme (22) miteinander verbunden sind, die die Schnittstelle der Arme von zwei aneinander liegenden Kreuzen verbinden, wobei zwei Verbindungsarme (22) selbst über Sammelarme (23) miteinander verbunden sind, die die Mitte der beiden Verbindungsarme verbinden.

## Claims

1. Device for grinding biological samples in order to extract DNA, RNA and proteins **characterised in that** it comprises several rotary pestles supported by a pestle-support block (1) and a mobile lower plate (5) free to move between a working position close to the pestle support block (1) and a rest position moved away from the pestle support block (1), each of the pestles comprising at least one duct for liquid distribution.

2. Device for grinding biological samples in order to extract DNA, RNA and proteins according to claim 1, **characterised in that** the lower plate (5) is provided with means of containing tubes (6) arranged in an arrangement conform with the pestles.

3. Device for grinding biological samples for extraction of DNA, RNA and proteins according to claim 1, **characterised in that** the lower plate (5) is provided with several little cups, arranged in an arrangement conform with the arrangement of the pestles.

4. Device for extraction of DNA, RNA and proteins from biological samples according to claim 1, 2 or 3, **characterised in that** it also comprises means of allowing liquid(s) to flow by gravity when the solenoid valves are opened.

5. Device for extraction of DNA, RNA and proteins starting from biological samples according to claim 1, 2 or 3, **characterised in that** it comprises pumps and means for purging catheters.

6. Device for extraction of DNA, RNA and proteins from biological samples according to claim 3, **characterised in that** the lower plate (5) comprises regulated heating devices.

7. Device for extraction of DNA, RNA and proteins from biological samples according to claim 1, **characterised in that** it also comprises a rigid board through which the pestle rods pass to cover each sample in its container during grinding in order to prevent cross-contamination.

8. Device for extraction of DNA, RNA and proteins from biological samples according to claim 1, **characterised in that** it also includes a cover plate composed of a superposition of plates (10 to 13), insert plates (11, 12) with a milling network reaching orifices for the supply of tubes (14).

9. Device for extraction of DNA, RNA and proteins from biological samples according to claim 8, **characterised in that** the cover comprises an upper plate (10) and two intermediate plates (11, 12) made of Teflon and a lower plate (13) made of silicone to make the tubes (14) leak tight.

10. Device for extraction of DNA, RNA and proteins from biological samples according to claim 9, **characterised in that** the millings are ducts forming a network for the distribution of fluids originating from a supply duct (15) leading to the opening of each of the tubes.

11. Device for the extraction of DNA, RNA and proteins from biological samples according to claim 10, **characterised in that** the ducts form series of cross pieces in the plate (12) composed of two arms (16, 17) each connecting two openings (18, 19), (20, 21) respectively, these cross pieces being connected in pairs by connecting arms (22) that join the intersection zone of the arms of the two adjacent cross-pieces, two connecting arms (22) themselves being connected by collecting arms (23) connecting the middle of the two connecting arms.
